# EUROPEAN PATENT SPECIFICATION

(11) **EP 0 336 620 B1**
(45) Date of publication and mention of the grant of the patent: **29.12.1993**
(21) Application number: 89302970.2
(22) Date of filing: 23.03.1989
(51) Int. Cl.: A61B 17/22

(54) **Apparatus for destroying calculuses**
Nierensteinzertrümmerer
Appareillage pour détruire les calcus

(30) Priority: 31.03.1988 JP 76096/88
(43) Date of publication of application: 11.10.1989
(73) Proprietor: KABUSHIKI KAISHA TOSHIBA, Kawasaki-shi, Kanagawa-ken 210 (JP)
(72) Inventor: Ishida, Akinori c/o Patent Division, Minato-ku Tokyo 105 (JP)
(74) Representative: Freed, Arthur Woolf

(56) References cited:
- EP-A- 0 081 051
- EP-A- 0 244 730
- DE-A- 3 621 935

## Description

The present invention relates to an apparatus for destroying calculuses of the type in which a calculus in a human body is disintegrated with the destroying energy externally applied.

A known apparatus for destroying calculuses is composed of destroying energy generator for generating destroying energy such as focused shock wave and focused ultrasonic wave, and an image processor for detecting a calculus existing in a human body and executing an appropriate processing for the purpose of breaking the detected calculus. In operation, it searches and detects a calculus in a human body, positions a focal point of an destroying energy at the detected calculus, and emits the destroying energy to the calculus to disintegrate. It is a rare case that the calculus can perfectly be disintegrated with the destroying energy of one time application. Normally the energy is repeatedly applied to the calculus. Meanwhile, the human body respires, causing a motion in the body. Therefore, if the focal point of the radiated energy is set at the calculus location, the calculus location, with progressing of the motion, will displace from the focal point. Consequently, the radiation hits another part in the body, which is located off the affected part, calculus. This possibly hurts another part of the body.

A measure for the above problem is disclosed in Japanese Patent Disclosure No. 63-158049. In the technique disclosed, a period of an end portion in an expiration duration is detected. During this period, the affected part is little displaced from the focal point of the radiation as set. The expiration end period is used for the positioning of the focus of the radiation at the affected part, and the generation of drive pulses for radiating the destroying energy that repetitively appear at a preset rate (repetitive frequency). In other words, the calculus is disintegrated only during that period. When the rate is, for example, 2Hz, viz., the destroying energy is radiated every 0.5 sec., a number of drive pulses that is required to disintegrate the calculus can be obtained during the expiration end period. If the rate is lower than 2Hz, for example, 0.5Hz, that is, if the pulse is generated every 2 sec., a number of drive pulses generated during the expiration end period is insufficient for obtaining the radiated energy enough to disintegrate the calculus. It is assumed that a number of breathings is 12 per minutes, a time of one breathings is 5 sec., an expiration duration is 3 sec., and the expiration end period is 2 sec. In this instance, if the rate is 0.5Hz, an extreme case may occur in which all pulses required for disintegrating the calculus are not generated during the expiration end period of 2 sec. Particularly, when the start time point of the expiration end period is not coincident with a time point of generating the first drive pulse, a number of pulses occurring during that period is reduced.

Another prior art is found in the catalogue of an extracorpreal urinary-passage stone lithotripsy device named "LITHOSTAR" manufactured by SIEMENS in West Germany. The catalogue describes a technique that shock waves are generated during a breathing gate in synchronism with the ECG in an electrocardiogram. The fact that the shock wave is generated in synchronism with a start point of the breathing gate, is not found in the catalogue, however. Therefore, there is the possibility the calculus moves, due to the breathing, from the focal point of the radiated breaking energy and hence the energy affects the tissue near the calculus.

EP-A-0244730 discloses an apparatus for destroying calculuses which has means for generating focused destroying energy. In this apparatus, means are provided for positioning the focus of the destroying energy at the location of a calculus in a human body. Further, means are provided for detecting the expiration end period of a breathing pattern of the human body. A control pulse is generated to control generation of the destroying energy with the control pulse beginning with the start point of the period. However, the released destroying energy does not necessarily start in coincidence with said start point.

Accordingly, an object of the present invention is to provide an apparatus for destroying calculuses which is capable of effectively generating drive pulses for destroying energy within a predetermined period regardless of the preset rate for generating the drive pulses, and hence is free from the another-part hurting problem caused by the mishitting of the destroying energy.

According to this invention, there is provided an apparatus for destroying calculuses comprising: means for generating focused destroying energy; means for positioning a focus of the destroying energy at a location of a calculus in a human body; and means for detecting a period during which the location of the calculus coincides with the focus of the destroying energy; and means for generating a first control pulse at a time point coincident with a start point of said period to control generation of the destroying energy, said first control pulse generating means being coupled with said period detecting means; characterized in that there is further provided means coupled to said focused destroying energy generating means, for generating, during said period, a train of control pulses to repeatedly generate destroying energy at a predetermined rate, additional and subsequent to the generation of said first control pulse; and said destroying energy starts at a time point coincident with a start point of said period.

With such an arrangement, an effective number of control pulses are generated during the said period regardless of the rate. Therefore, the problem that the destroying energy mishits another part located off the affected part, calculus and adversely affects there is successfully solved. Additionally, a speedy treatment can be realized.

This invention can be more fully understood from the following detailed description when taken in conjunction with the accompanying drawings, in which:
Fig. 1 is a block diagram showing an apparatus for destroying calculuses according to an embodiment of the present invention;
Fig. 2 is a block diagram of a detector for detecting an expiration end period, which is used in the apparatus of Fig. 1;
Figs. 3A through 3C are waveforms of signals at output portion of respective unit in the detector of Fig. 2;
Figs. 4D through 4H are a timing chart useful in explaining the operation of the detector of Fig. 2;
Fig. 5 is a block diagram showing an apparatus for destroying calculuses according to another embodiment of the present invention; and
Fig. 6 shows waveforms for explaining the operation of a coincidence detect circuit for detecting a coincidence of a location of a calculus with a focus of ultrasonic wave energy.

An apparatus for destroying calculuses for disintegrating a calculus in a human body using a shock wave energy as a destroying energy according to the present invention, will be described with reference to the accompanying drawings.

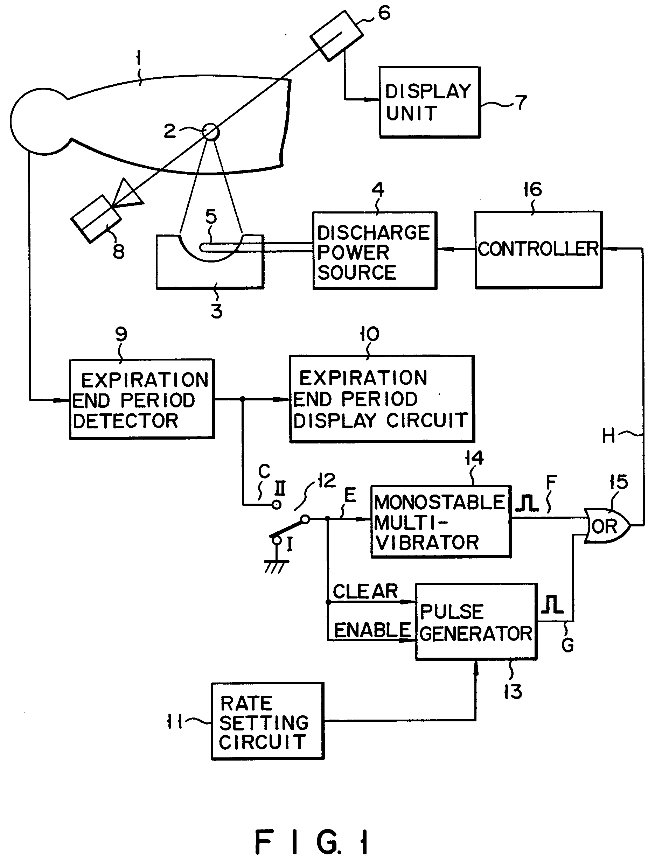

Fig. 1 shows an arrangement of an apparatus for destroying calculuses according to an embodiment of the present invention. As shown, an ellipsoidal reflector 3 is so arranged that its focus is positioned at a calculus 2 in a human body 1. In the treatment, charges stored in a discharge power source 4 are discharged through a spank gap 5 located at a first focus point of the ellipsoidal reflector 3. A shock wave energy generated there is reflected by the ellipsoidal reflector 3, and is projected to the calculus 2 as a second focus of the ellipsoidal reflector. The focus of the ellipsoidal reflector 3 is positioned at a location of the calculus 2 in the human body 1, while seeing a display screen of a display unit 7 which presents the information of the innards of the human body as detected by an X-ray detector 6. An X-ray tube 8 and the detector 6 are disposed oppositely with the human body 1 intervening therebetween. An operator moves the human body 1 while seeing the display, and when the calculus 2 is positioned at the center of the display, the human body 1 is fixed. The display unit 7 is so arranged that the center of the display screen indicates the second focus of the ellipsoidal reflector 3. Accordingly, when the center of the display coincides with the calculus displayed, the destroying energy is radiated toward the calculus, to destroy the calculus.

An expiration end period detector 9 detects a expiration end period from a breathing period of the human body 1. A motion of the human body due to his breathing, viz., a movement of the calculus, is relatively small during the expiration end period. During this period, therefore, the positioning of the calculus to the focus is performed and the timing of generating an shock wave energy is decided, as already stated.

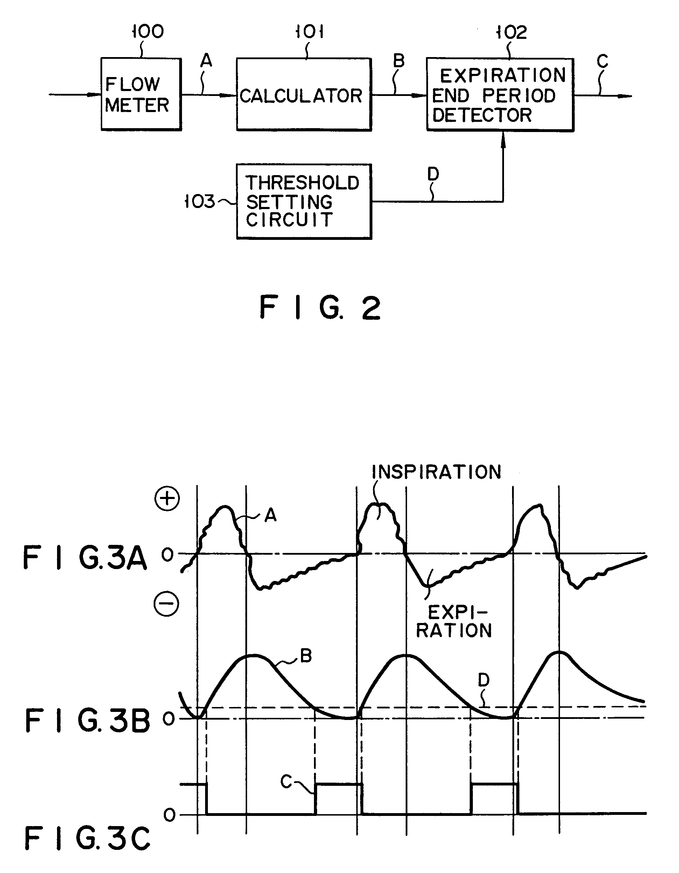

A detailed arrangement of the expiration end period detector 9 will be described with reference to Figs. 2 and 3. In Fig. 2, a flow meter 100 measures a respiratory flow caused by a breathing, through a transducer (not shown) attached to a patient. A waveform of respiratory flow as denoted as A is illustrated in Fig. 3A. In the figure, (+) indicates a variation of the inspiratory flow and (-) indicates a variation of the expiratory flow. The result of respiratory flow measurement is supplied to a respiratory volumen calculator 101 which integrates the waveform A to obtain a waveform B representing an amount of respiratory volume in Fig. 3B. An expiration end period detector 102 compares a signal of the waveform B with a signal representative of a threshold value D (Fig. 3B), which is set by a threshold value setting circuit 103. A period that the waveform B is smaller than the threshold value D is produced in terms of an expiration period end signal C.

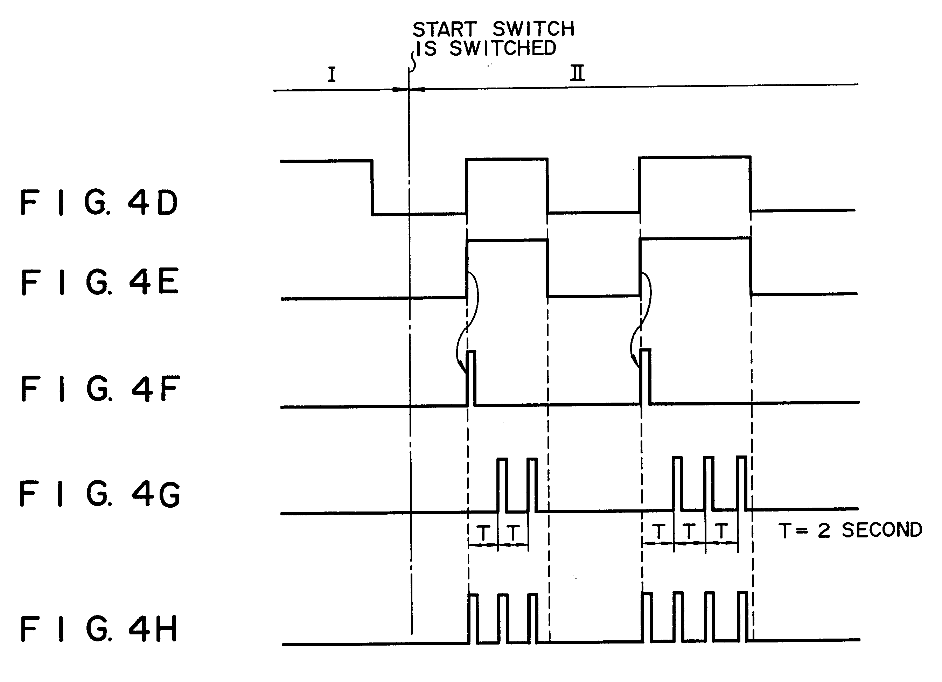

The operation of the apparatus for destroying calculuses thus arranged will be described in connection with the handling of the apparatus. An expiration end period in a breathing of a patient is detected by the expiration end period detector 9. Receiving the detect result, the display unit 10 presents the expiration end period in the form of a periodic tone or an illumination to a patient. An operator positions a calculus of a patient at the second focus of the ellipsoidal reflector 3, viz, the center of a display screen of the display unit 7. This is done while he seeing an X-ray image on the display, and hearing the periodic tone or seeing the illuminating representing the expiration end period. The positioning thus obtained is correct during the expiration end period even when the calculus location is moved by his breathing. The operation to follow is to disintegrate the calculus. To this end, a rate setting circuit 11 is first operated to set a frequency at 0.5Hz, for example. This figure indicates a low rate, and that shock wave energy is radiated every 2 sec. Then, a start switch 12 is switched over from position I to position II. Reference is made to Figs. 4D to 4H. At the position I, the enable terminal and clear terminal of a pulse generator 13, and the input terminal of a monostable multivibrator 14 are coupled with ground. In this state, the pulse generator 13 is free from the calculus disintegrating operation. When the switch 12 is turned to the position II manually or automatically, all the above terminals are connected to the expiration end period detector 9. When a expiration end period (the positive portion of the waveform C in Fig. 3C) is detected, the multivibrator 14 generates a first pulse (F) as shown in Fig. 4F. This pulse (F) is applied as a first pulse of pulses (H) (Fig. 4H) to a controller 16, by way of an OR gate 15. The pulse generator 13 is enabled, to generate pulses (G) (Fig. 4G) recurring at the rate (0.5Hz, appearing every 2 sec.) as already set by the rate setting circuit 11. The pulses (G) also pass the OR gate 15 and, succeeding to the pulse (F), go in the form of the pulses (H) to the controller 16. The above operation is performed during the expiration end period. When this period terminates, the pulse generator 13 is disabled, and a counter for the pulse outputting is also cleared, and waits for the next expiration end period. Thus, during the expiration end period, a possibly longest train of pulses (H) is applied at the set rate to the controller 16. In turn, it causes the discharge power source 4 to discharge the stored charges through the spark gap 5. The resultant shock wave energy is applied to the affected part, calculus, and disintegrates it. Then, the operator confirms by using the display unit 7 that the calculus is disintegrated, and returns the switch 12 to the position I. Here, the treatment operation by using the apparatus for destroying calculuses according to this invention ends.

In the above embodiment, a combination of the monostable multivibrator 14, pulse generator 13, and OR gate 15 is used for forming the pulse train of the set rate. If required, a train of pulses appearing at the start point of the expiration end period may be formed by a combination of a microcomputer and a timer.

Additionally, it is evident that a focused ultrasonic energy may is substituted for the shock wave energy.

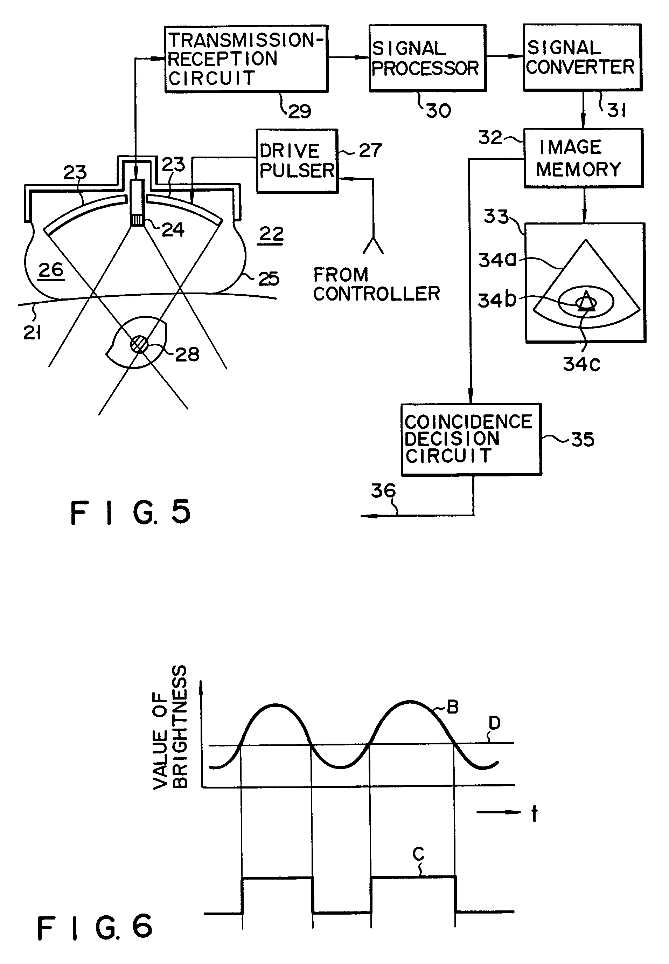

An embodiment of an apparatus for destroying calculuses according to the present invention, which uses a focused ultrasonic energy as the destroying energy, will be described with reference to Figs. 5 and 6.

As shown, an applicator 22 is used in contact with the surface of a human body 21. Within the applicator 22, a disintegrating transducer 23 for breaking a calculus and imaging ultrasonic probe 24 for imaging the calculus are disposed as shown. The imaging ultrasonic probe 24 is located at the center of the disintegrating transducer 23. The applicator 22 is hermetically sealed by a membrane 25. The applicator 22 thus formed is filled with a liquid of an appropriate acoustic impedance, for example, water 26. When the applicator 22 is placed in close contact with the surface of the human body 21, the focused ultrasonic energy is allowed to radiate the innards of the human body 21. The disintegrating transducer 23 is coupled with a drive pulser 27 by way of a cable. The drive pulser 27 is driven with a control signal derived from a controller (not shown), to radiate a focused ultrasonic energy into the human body 21.

The imaging ultrasonic probe 24 is excited so as to make a sector scan, for example. An echo signal resulting from the sector scan is applied to a transmission/reception circuit 29. The output signal from the circuit 29 is applied to a signal processor 30 where it is amplitude detected. The detected signal is applied in the form of a digital video signal to a converting system 31. The converting system 31, made up of a frame memory, line memory, etc., applies an appropriate signal processing to he digital video signal. The output signal from the converting system 31 is temporarily stored in an image memory 32, and is applied to a TV monitor 33. Receiving the signal, the image memory 32 displays an acoustic domain image 34a in a sector fashion, an image 34b including a kidney, calculus, etc., and a focal marker 34c representing a focus of the ultrasonic energy transmitted from the disintegrating transducer 23.

The sector image (B-mode image) 34a on the TV monitor presents a calculus in the human body. The calculus image has a high acoustic impedance, and therefore reflects the ultrasonic wave applied in a high intensity. The result is that the image 34a is brighter than its peripheral organic image. The focal marker 34c is fixed on the monitor screen. The reason for this is that the disintegrating transducer 23 and imaging ultrasonic probe 24 are fixedly disposed as shown. Therefore, to position the calculus image 34b at the focal marker 34, all one has to do is to move the applicator 22 so that both are coincident with each other in position. To check if the focal marker 34c coincides with the calculus image 34b, a coincidence decision circuit 35 is provided. The decision circuit 35 works out a value of brightness of the acoustic domain image (B-mode image) contained in the focal marker 34c region, which is stored in the image memory 32, and outputs a coincidence-degree signal 36 every 5 sec., for example. More exactly, the coincident decision circuit 35 searches value of brightness B in excess of a predetermined value of brightness D and produces the values B in the form of the coincidence degree signal C. The duration of the signal C is used as the expiration end period. During this period, the disintegrating transducer is allowed to radiate the ultrasonic wave toward the calculus. The ultrasonic wave radiated, therefore, exactly hits the affected part, without hurting the tissue near the calculus in the human body.

## Claims

1. An apparatus for destroying calculuses comprising:
means (4, 5; 23, 27) for generating focused destroying energy;
means for positioning a focus of the destroying energy at a location of a calculus in a human body; and
means (9; 29-35) for detecting a period during which the location of the calculus coincides with the focus of the destroying energy; and
means (14) for generating a first control pulse at a time point coincident with a start point of said period to control generation of the destroying energy, said first control pulse generating means being coupled with said period detecting means;
characterized in that there is further provided
means (13) coupled to said focused destroying energy generating means, for generating, during said period, a train of control pulses to repeatedly generate destroying energy at a predetermined rate, additional and subsequent to the generation of said first control pulse; and
said destroying energy starts at a time point coincident with a start point of said period.

2. An apparatus for destroying calculuses according to claim 1, characterized in that said detecting means (9) detects an expiration end period of a breathing pattern of a human body.

3. An apparatus for destroying calculuses according to claim 1, characterized in that said detecting means (9) detects the period during which a value of brightness of the image of a calculus exceeds a predetermined value.

4. An apparatus for destroying calculuses according to claim 1, characterized in that said means (14) for generating a first pulse to drive said radiated energy is a monostable multivibrator.

## Patentansprüche

1. Steinzertrümmerungsgerät mit
einer Einrichtung (4, 5; 23, 27) zum Erzeugen einer fokussierten bzw. gesammelten Zertrümmerungsenergie;
einer Einrichtung zum Positionieren eines Fokus der Zertrümmerungsenergie an einer Stelle eines Steins in einem menschlichen Körper; und
einer Einrichtung (9; 29-35) zum Erkennen einer Periode, in der die Stelle des Steins mit dem Fokus der Zertrümmerungsenergie zusammenfällt; und
einer Einrichtung (14) zum Erzeugen eines ersten Steuerimpulses zu einem Zeitpunkt, der mit einem Startpunkt der Periode zusammenfallend ist, um die Erzeugung der Zertrümmerungsenergie zu steuern, wobei die Einrichtung zur Erzeugung des ersten Steuerimpulses mit der Periodenerkennungseinrichtung gekoppelt ist; dadurch gekennzeichnet,
daß weiter eine Einrichtung (13) bereitgestellt ist, die mit der Einrichtung zur Erzeugung der fokussierten Zertrümmerungsenergie gekoppelt ist, um während der Periode eine Reihe von Steuerimpulsen zu erzeugen, um die Zertrümmerungsenergie zusätzlich und nach der Erzeugung des ersten Steuerimpulses wiederholt mit einer vorbestimmten Rate zu erzeugen; und daß die Zertrümmerungsenergie zu einem Zeitpunkt beginnt, der mit einem Startpunkt der Periode zusammenfallend ist.

2. Steinzertrümmerungsgerät nach Anspruch 1, dadurch gekennzeichnet, daß die Erkennungseinrichtung (9) eine Ausatmungsendeperiode eines Atmungsmusters eines menschlichen Körpers erkennt.

3. Steinzertrümmerungsgerät nach Anspruch 1, dadurch gekennzeichnet, daß die Erkennungseinrichtung (9) die Periode erkennt, in der ein Wert der Helligkeit des Bilds eines Steins einen vorbestimmten Wert überschreitet.

4. Steinzertrümmerungsgerät nach Anspruch 1, dadurch gekennzeichnet, daß die Einrichtung (14) zum Erzeugen eines ersten Impulses zum Treiben der abgestrahlten Energie ein monostabiler Multivibrator ist.

## Revendications

1. Appareil de destruction de calculs, comprenant :
un dispositif (4, 5 ; 23, 27) destiné à créer de l'énergie destructrice focalisée,
un dispositif de positionnement d'un foyer de l'énergie destructrice à l'emplacement d'un calcul présent dans un corps humain, et
un dispositif (9 ; 29-35) de détection d'une période pendant laquelle l'emplacement du calcul coïncide avec le foyer de l'énergie destructrice, et
un dispositif (14) destiné à créer une première impulsion de commande à un moment coïncidant avec un moment initial de ladite période afin que la création de l'énergie destructrice soit réglée, ce dispositif générateur d'une première impulsion de commande étant couplé au dispositif de détection de période,
caractérisé en ce qu'il comporte en outre :
un dispositif (13) couplé au dispositif générateur d'énergie destructrice focalisée et destiné à créer, pendant ladite période, un train d'impulsions de commande pour la création répétée d'énergie destructrice à une fréquence prédéterminée, en plus de la création de la première impulsion de commande et après cette création, et
l'énergie destructrice commence à un moment qui coïncide avec le moment initial de ladite période.

2. Appareil de destruction de calculs selon la revendication 1, caractérisé en ce que le dispositif de détection (9) détecte une période de fin d'expiration d'un cycle respiratoire d'un corps humain.

3. Appareil de destruction de calculs selon la revendication 1, caractérisé en ce que le dispositif de détection (9) détecte la période au cours de laquelle une valeur de la luminosité de l'image d'un calcul dépasse une valeur prédéterminée.

4. Appareil de destruction de calculs selon la revendication 1, caractérisé en ce que le dispositif (14) générateur d'une première impulsion de pilotage de l'énergie rayonnée est un multivibrateur monostable.
